(19) Europäisches Patentamt
European Patent Office
Office européen des brevets

(11) **EP 0 865 802 B1**

(12) **EUROPÄISCHE PATENTSCHRIFT**

(45) Veröffentlichungstag und Bekanntmachung des Hinweises auf die Patenterteilung:
**22.12.2004 Patentblatt 2004/52**

(51) Int Cl.$^7$: **A61N 1/368**

(21) Anmeldenummer: **98250076.1**

(22) Anmeldetag: **03.03.1998**

(54) **Vorrichtung zur Bestimmung der AV-Überleitungszeit**

Device for determining the AV delay

Appareil pour la détermination du délai AV

(84) Benannte Vertragsstaaten:
**CH DE FR LI NL**

(30) Priorität: **03.03.1997 DE 19711058**

(43) Veröffentlichungstag der Anmeldung:
**23.09.1998 Patentblatt 1998/39**

(73) Patentinhaber: **BIOTRONIK Mess- und Therapiegeräte GmbH & Co Ingenieurbüro Berlin**
**12359 Berlin (DE)**

(72) Erfinder: **Hartung, Wolfgang, Dr.**
**39114 Magdeburg (DE)**

(74) Vertreter: **Eisenführ, Speiser & Partner**
**Patentanwälte Rechtsanwälte**
**Spreepalais am Dom**
**Anna-Louisa-Karsch-Strasse 2**
**10178 Berlin (DE)**

(56) Entgegenhaltungen:
**EP-A- 0 607 951     US-A- 5 334 222**
**US-A- 5 368 040     US-A- 5 626 623**
**US-A- 5 836 987**

**Beschreibung**

Die Erfindung betrifft eine Vorrichtung zur Bestimmung der AV-Überleitungszeit bzw. AV-Verzögerung im Herzen, mit einer im Herzen angeordneten Signalaufnehmeranordnung (EA, EV, P; 31a-d, 32af) mit der eine natürliche Herzaktion erfasst werden kann, mit mindestens einem Signalausgang und einer Verarbeitungseinheit (2; 33) mit mindestens einem Signaleingang, der mit dem Signalausgang der Signalaufnehmeranordnung verbunden ist, zur Berechnung der natürlichen AV-Überleitungszeit aufgrund der erfassten Herzaktion.

Die Erfindung betrifft einen Herzschrittmacher mit einer solchen Vorrichtung.

Die Einstellung der Zeitspanne zwischen einer natürlichen oder stimulierten Vorhofaktion und dem Stimulationsimpuls zur Stimulation einer Kammeraktion, die sogenannte AV-Verzögerung (AV delay), hat erhebliche Bedeutung für die Optimierung der Schrittmacherfunktion unter hämodynamischen Gesichtspunkten sowie im Zusammenhang mit der Verhinderung von schrittmacherinduzierten Tachykardien, vgl. M. Schaldach, Electrotherapy of the Heart - Technical Aspects in Cardiac Pacing, Springer Verlag Berlin Heidelberg 1992, S. 58 bis 60.

In den letzten Jahren sind daher Schrittmacher bekannt geworden, bei denen die früher fest eingestellte AV-Verzögerung in Abhängigkeit von Parametern, die in gewisser Weise den aktuellen Zustand eines speziellen Patienten reflektieren, verändert werden kann. Dabei kommt als Bezugsbasis zunächst die aktuelle Herz- bzw. Stimulationsrate in Frage, wobei die AV-Verzögerung mit zunehmender Stimulationsrate verringert wird - vgl. a.a.O. Fig. 30 auf S. 60.

In EP 0 450 387 A2 wird eine Anordnung zur automatischen Einstellung der AV-Verzögerung bei einem Zweikammer-Schrittmacher unter Berücksichtigung von (patientenspezifischen) intraatrialer Verzögerungszeiten beschrieben.

In EP 0 494 487 A2 wird ein AV-sequentieller Zweikammer-Schrittmacher mit automatischer Programmierung der AV-Verzögerung beschrieben, bei dem die Berücksichtigung schrittmacherbedingter interatrialer und interventrikulärer Überleitungszeiten angestrebt wird. Hier wird in einer dem eigentlichen Schrittmachen vorgeschalteten Untersuchungsphase zunächst die natürliche Herzrate und - als Zeitdifferenz entweder zwischen der natürlichen P-Welle oder einem atrialen Schrittmacher-"Spike" und der natürlichen R-Welle oder einem ventrikulären Schrittmacher-"Spike" (wobei alle Signale über die Schrittmacherelektroden erfaßt werden) - eine natürliche AV-Verzögerung bestimmt.

Aus der US-A- 5 334 222 ist ein Schrittmacher bekannt, welcher einen Druckaufnehmer aufweist und eine AV-Überleitungszeit aus einem Auswertungsergebnis eines Druckverlaufes ermitteln kann.

Da mit dieser Anordnung während der Untersuchungsphase nicht normal stimuliert werden kann, wird ein aus einigen (beispielsweise 4) natürlichen Herzaktionen gewonnener Wert der AV-Verzögerung einer sich anschließenden, längerwährenden Stimulationsphase (von beispielsweise 100 Impulsen) zugrundegelegt. Eine wirklich zeitnahe Bestimmung und Verwendung der jeweils aktuellen AV-Verzögerung zur Steuerung des Schrittmachers ist damit nicht möglich, so daß der Schrittmacher im Hinblick auf plötzliche Zustandsänderungen, etwa einsetzende oder aufhörende Belastungen, des Patienten hämodynamisch nicht optimal arbeiten kann.

Dieser Nachteil haftet natürlich auch den bekannten Verfahren zur Bestimmung der AV-Überleitungszeit aus einem Überflächen-EKG, echokardiographischen Verfahren o.ä. an, da derartige Untersuchungen im Alltagsleben des Patienten nicht permanent durchgeführt werden können.

Der Erfindung liegt die Aufgabe zugrunde, eine Vorrichtung der eingangs genannten Gattung, die - insbesondere in Verbindung mit einem Herzschrittmacher - zum permanenten Einsatz am Patienten geeignet ist, sowie einen eine solche Vorrichtung aufweisenden Schrittmacher anzugeben.

Diese Aufgabe wird durch eine Vorrichtung bzw. einen Schrittmacher der eingangs genannten Art gelöst, bei welcher die Signalaufnehmeranordnung eine Druck- bzw. Bewegungsaufnehmeranordnung zur Erfassung von Bewegungen der Trikuspidalklappe und/ oder intrakardialer Druckänderungen als Ausdruck der Herzaktion aufweist, welche benachbart zur Trikuspidalklappe im rechten Vorhof und/oder in der rechten Herzkammer angeordnet werden kann, wobei die Druckaufnehmeranordnung mehrere Druck- bzw. Bewegungsaufnehmer umfasst, welche in verschiedenen Positionen relativ zur Trikuspidalklappe angeordnet werden können und die jeweils einen Signalausgang aufweisen, und dass die Verarbeitungseinheit eine der Anzahl der Druckaufnehmer entsprechende Anzahl von Signaleingängen sowie Mittel zur Auswertung der von den mehreren Druck- bzw. Bewegungsaufnehmern empfangenen Signale unter Berücksichtigung ihrer jeweiligen zeitlichen Verzögerung aufweist.

Die Erfindung schließt den Gedanken ein, ausgehend von der Korrelation zwischen charakteristischen Erregungszuständen des leitfähigen Herzgewebes und dem jeweiligen mechanischen Zustand des Herzens eine zeitnahe bzw. Echtzeit-Bestimmung der natürlichen AV-Überleitungszeit auf mechanischer Grundlage vorzunehmen. Sie nutzt dabei die Erkenntnis aus, daß charakteristische Signale im Oberflächen-EKG bzw. korrespondierende Änderungen des His-Bündel-Potentials, die bei der Aufnahme eines intrakardialen EKG erfaßt werden und die zur Bestimmung der AV-Überleitungszeit herangezogen werden können, ein mechanisches Äquivalent in den Klappenbewegungen insbesondere der Trikuspidalklappe bzw.

intrakardialen Druckschwankungen haben. Es konnte insbesondere festgestellt werden, daß synchron zur R-Welle und zum Beginn der P-Welle Druckspitzen auftreten.

Zur Störunterdrückung umfasst die Druck- bzw. Bewegungsaufnehmeranordnung mehrere, in verschiedenen Stellungen relativ zur Trikuspidalklappe angeordnete Druck- bzw. Bewegungsaufnehmer, die jeweils einen Signalausgang aufweisen, und die Verarbeitungseinheit eine der Anzahl der Druck- bzw. Bewegungsaufnehmer entsprechende Anzahl von einzelnen Signaleingängen sowie Mittel zur gemeinsamen Auswertung der von den Aufnehmern empfangenen Signale unter Berücksichtigung ihrer jeweiligen zeitlichen Verzögerung aufweist.

Mit einer solchen Anordnung lassen sich mehrere, im wesentlichen gleichartige, Druck- oder Bewegungssignale gewinnen, die infolge der begrenzten Geschwindigkeit der intrakardialen Druckausbreitung zeitlich gegeneinander versetzt sind und die etwa mittels einer Signalakkumulation (nach Elimination des Zeitversatzes) oder Korrelationsanalyse zur Erhöhung des Signal-Rausch-Verhältnisses genutzt werden können.

In einer zweckmäßigen Ausbildung ist mindestens ein Druck- bzw. Bewegungsaufnehmer im Vorhof und mindestens einer in der Herzkammer angeordnet.

Eine alternativ oder zusätzlich zur vorgenannten Variante anwendbare Ausführung mit verbesserter Störunterdrückung ist dadurch gekennzeichnet, daß der Verarbeitungseinheit Sensormittel zur Erfassung eines spontanen oder induzierten Vorhofsignals mit einem Signalausgang zugeordnet sind, der mit einem entsprechenden Signaleingang der Verarbeitungseinheit verbunden ist, und daß sie Zeitgebermittel und Schaltmittel zur Sperrung des mit der Druck- bzw. Bewegungsaufnehmeranordnung verbundenen Signaleingangs zur Vorgabe eines Zeitfensters für die Erfassung der Eingangssignale der Druck- bzw. Bewegungsaufnehmeranordnung aufweist. Dieses Zeitfenster wird mit einer atrialen Signalwahrnehmung oder Stimulation geöffnet und bleibt nur für die Zeitspanne, in der mit dem systolischen Druckanstieg gerechnet werden muß, d.h. ca. 250 300 ms, offen. Außerhalb dieses Zeitraumes auftretende Druckschwankungen, die ohnedies für die vorgesehene Messung nur Störsignale darstellen können, werden somit von vornherein von jeder Verarbeitung ausgeschlossen.

In einer hinsichtlich der Auswertungsmöglichkeiten besonders variablen Ausführung weist die Verarbeitungseinheit Mittel zur Diskriminierung von Bewegungs- und Druckanteilen im Signal des Druck- bzw. Bewegungsaufnehmers, etwa mindestens ein niederfrequentes Bandpaßfilter, auf. Die eigentlichen Auswertungsstufen sind in ihrem Aufbau und/oder ihrer Programmierung dann auf den jeweils spezifischen Informationsgehalt der Klappenbewegungsabläufe bzw. der von Bewegungseinflüssen bereinigten intrakardialen Druckänderungen zugeschnitten.

Zweckmäßigerweise ist die Aufnehmeranordnung auf einem Herzkatheter angeordnet. Sie kann vom Fachmann entsprechend bekannten Aufnehmern zur intraarteriellen oder intrakardialen Blutdruckmessung ausgeführt werden. In vorteilhafter Ausbildung weist sie etwa einen die Bewegung oder Verformung einer Druckaufnahmefläche auf elektrischem Wege erfassenden Sensor, insbesondere einen piezoelektrischen Drucksensor, oder einen diese auf optischem Wege, insbesondere durch Ablenkung oder Änderung der Transmission eines auf die Fläche gerichteten, faseroptisch zum Meßort übertragenen Lichtbündels, erfassenden Sensor auf. Bei einer Anordnung mit mehreren Drucksensoren werden auf dem Herzkatheter mehrere, jeweils durch voneinander getrennte Mantelflächenabschnitte des Herzkatheters gebildete oder mit solchen verbundene, Druckaufnahmeflächen angeordnet sein.

Um eine von Wandkontakten unbeeinflußte Druck- bzw. Bewegungserfassung zu sichern, ist der Herzkatheter zweckmäßigerweise mit einer solchen Biegesteifigkeit aufgebaut und ggfs. solchen vorgeprägten Krümmung im Längsverlauf versehen, daß er im wesentlichen ohne Kontakt der Druckaufnahmeflächen mit den Herzinnenwandungen freiliegend im Vorhof und/ oder der Herzkammer angeordnet werden kann.

Zur Bestimmung der AV-Verzögerung gegenüber einem wahrgenommenen natürlichen Vorhofsignal ist mindestens eine Elektrode zur Aufnahme eines Herzaktionspotentials im Atrium vorzusehen, die mit einem entsprechenden Signaleingang der Verarbeitungseinheit verbunden ist, und die Verarbeitungseinheit umfaßt Mittel zur Bestimmung des Zeitabstandes zwischen einem aus dem Herzaktionspotential abgeleiteten und einem von der Druck- bzw. Bewegungsaufnehmeranordnung gelieferten Signal. Derartige Mittel sind ebenso wie geeignete Elektrodenaufbauten als solche von herkömmlichen Schrittmachern bekannt. Bei Einsatz von zwei oder mehr Elektroden ist in vorteilhafter Weise eine differentielle Erfassung des Vorhofsignals möglich.

Zur Bestimmung der AV-Verzögerung gegenüber einem künstlichen Reizimpuls ist eine Vorrichtung zur Erzeugung des Reizimpulses und zu dessen Übertragung an den Vorhof vorzusehen, die einen Signalausgang aufweist, der mit einem entsprechenden Signaleingang der Verarbeitungseinheit verbunden ist, und die Verarbeitungseinheit umfaßt Mittel zur Bestimmung des Zeitabstandes zwischen einem abgegebenen Reizimpuls und einem von der Druck- bzw. Bewegungsaufnehmeranordnung gelieferten Signal. Die Vorrichtung zur Erzeugung des Reizimpulses kann natürlich der entsprechende Teil eines herkömmlichen Schrittmachers sein, und zur Übertragung wird ggfs. eine vorhandene Vorhofelektrode genutzt.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Fig. 1 ein Blockschaltbild eines extern programmierbaren AV-sequentiellen Zweikammerschrittmachers mit zugehörigem Herzkatheter, mit dem eine Ausführungsform der Erfindung realisiert wird,

Fig. 2 eine vergleichende Darstellung der Signalverläufe bei verschiedenen Meßverfahren zur Erfassung von Herzaktionen,

Fig. 3a eine schematische Gesamtansicht eines bei der Ausführung der Erfindung verwendeten Herzkatheters mit Elektrodenabschnitten zur Erfassung eines intrakardialen EKG und mehreren Druckaufnehmern im in das Herz eingeführten Zustand und

Fig. 3b Darstellungen von zeitlichen Signalverläufen der in Fig. 3a dargestellten Druckaufnehmer im Vergleich mit dem Signalverlauf eines parallel aufgenommenen Oberflächen-EKG.

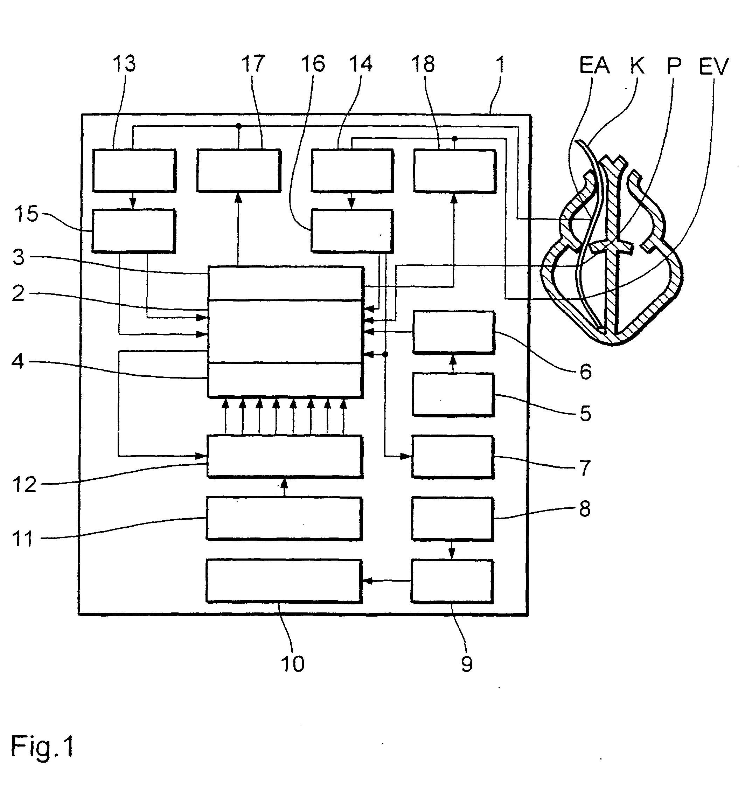

Der In Fig. 1 gezeigte Zweikammerschrittmacher 1 hat einen an sich im wesentlichen bekannten Aufbau, der daher nachfolgend nur knapp skizziert wird: Kernstück ist eine Steuereinheit 2, der eine Schaltung 3 zum "Run-Away"-Schutz und ein Programmdecoder 4 unmittelbar zugeordnet sind. Eine Batterie 5 mit nachgeschaltetem EOL-Indikator 6 versorgt den Schrittmacher mit Energie. Auf die Aktivierung durch einen Reed-Schalter 7 hin wird über eine Empfängerspule 8, einen Empfänger 9, einen Programmverstärker 10 und eine Sicherheitsschaltung 11 zur Programmüberprüfung ein Programmregister 12 geladen, aus dem durch die Steuereinheit Programmdaten abgefragt werden können.

Dem Schrittmacher 1 ist ein Herzkatheter K zugeordnet, das eine (rechts-)atrial und eine (rechts-)ventrikulär angeordnete Elektrodenannordnung EA bzw. EV sowie eine Druckaufnehmeranordnung P aufweist. Über die Elektrodenanordnungen EA und EV werden Herzaktionspotentiale erfaßt und über einen Eingangsverstärker 13 für atriale Signale und einen Eingangsverstärker 14 für ventrikuläre Signale mit jeweils nachgeschalteter Störerkennungsschaltung 15 bzw. 16 ebenso der Steuereinheit 2 zugeführt wie Signale von der Druckaufnehmeranordnung P, die intrakardiale Druckänderungen infolge der Bewegungen der Herzklappen reflektieren. Die genannten Eingangssignale werden in der Steuereinheit 2 einer Verarbeitung unterzogen und zur Steuerung des Schrittmachers entsprechend dem aktuellen Herzleistungsbedarf des Patienten herangezogen.

Mit entsprechenden Parametern erzeugte Reizimpulse werden in jeweils einer Impulserzeuger- und Ausgangsverstärkerschaltung 17 bzw. 18 erzeugt und an die Elektrodenanordnungen EA bzw. Ev geliefert und von diesen schließlich an das reizbare Herzgewebe übertragen.

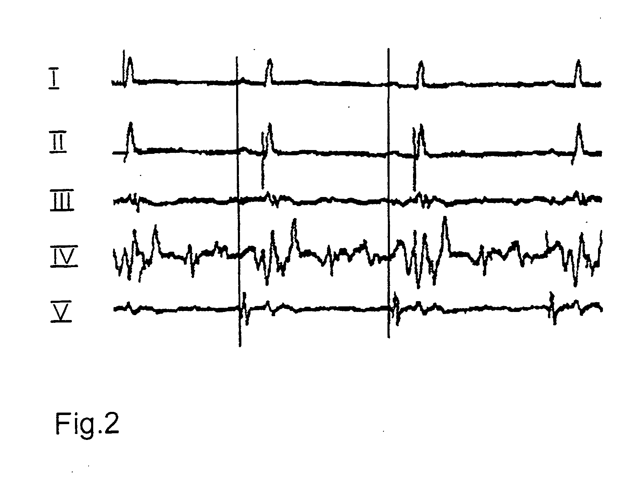

Fig. 2 ist eine vergleichende Darstellung der Signalverläufe bei verschiedenen Meßverfahren zur Erfassung von Herzaktionen, die im Zusammenhang mit der Bestimmung der AV-Überleitungszeit bzw. -Verzögerung zu beachten ist.

Der Signalverläufe I und II sind Oberflächen-EKG-Diagramme und Kurve III zeigt ein His-Bündel-Elektrogramm, d.h. das Ergebnis einer intrakardialen Registrierung der Herzaktionspotentiale. Mit "A" und "V" sind jeweils ein atrialer bzw. ventrikulärer Stimulationsimpuls bezeichnet.

Die Kurve IV zeigt Registrierungen mittels eines als "Piezo cath" angegebenen, intrakardial (speziell rechtatrial) plazierten Drucksensors. Es ist zu erkennen, daß in Synchronität zu den Herzaktionen nachweisbare Druckschwankungen auftreten, wobei der Kurvenverlauf charakteristische Punkte - etwa die mit "P1" und "P2" und jeweils einem Pfeil bezeichneten Punkte - zeigt. P1 steht mit dem Beginn der Öffnung der Trikuspidalklappe und P2 mit dem Ende der Systole in Zusammenhang.

Beide Punkte P1 und P2 können als Triggerpunkte für die Bestimmung der AV-Überleitungszeit bzw. -Verzögerung anstelle der R-Zacke des EKG bzw. einer entsprechenden Registrierung im His-Bündel-Elektrogramm (HBE) verwendet werden, womit die Druckmessung eine elektrografische Messung zur Bestimmung der AV-Verzögerung - insbesondere bei einer Echtzeitbestimmung zur Anpassung einer entsprechenden Schrittmachereinstellung an die aktuellen hämodynamischen Bedürfnisse des Patienten - ersetzen kann.

Ausgehend von P2 als Triggerpunkt (TgP) kann etwa mit der Beziehung

$$AVdelay_n = aST_n + (aST_{n-1} \cdot TgP_{n-1} - x \text{ ms})$$

mit

$aST_n$ = n-te atriale Stimulation/n-tes Sensing,

$aST_{n-1}$ = (n-1)-te atriale Stimulation/(n-1)-tes Sensing,

$TgP_{n-1}$ = Triggerpunkt bei der (n-1)-ten atrialen Stimulation/dem (n-1)-ten Sensing,

x ms = Zeitspanne, die die optimale Stimulation des Ventrikels vor dem Triggerpunkt liegt,

eine Echtzeit-AV-Bestimmung vorgenommen werden (x = gesuchte Größe).

Durch die Erfassung und entsprechende Auswertung von Signalen mehrerer, in unterschiedlichem Abstand von der Trikuspidalklappe - insbesondere hintereinander auf einem Katheter - angeordneter Drucksensoren ist eine zeitliche und lokale Differenzierung von Störsignalen möglich. Dazu ist eine Signalverarbeitung unter Zuhilfenahme von Signalakkumulations- und/oder Korrelationsverfahren anwendbar.

Eine weitere Möglichkeit der Störunterdrückung ist der Betrieb des Drucksensors bzw. der Drucksensoren in einem Zeitfenster (von beispielsweise 250 bis 300 ms), das mit der atrialen Signalwahrnehmung bzw. Stimulation öffnet.

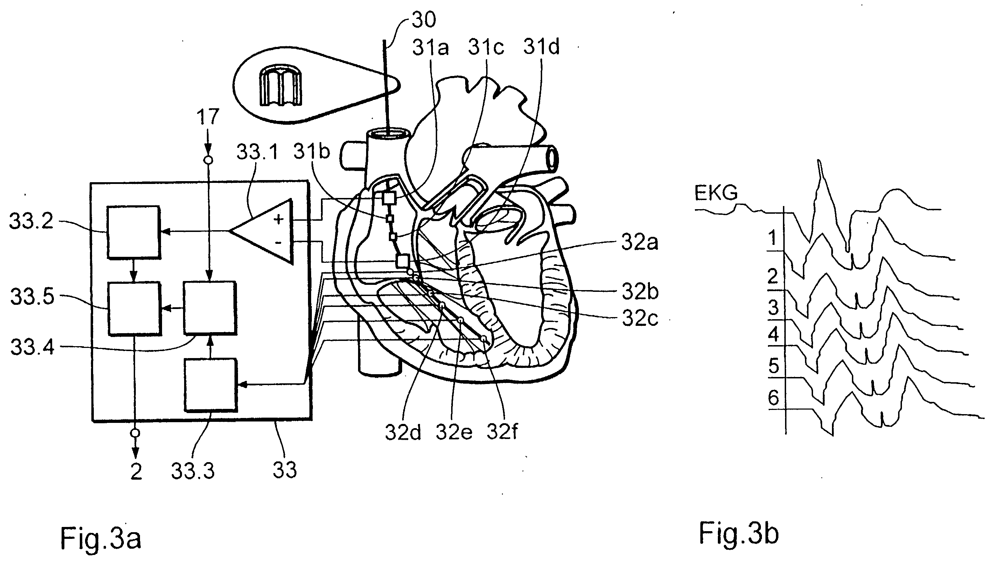

Fig. 3a ist eine stark schematische Gesamtansicht eines in ein Herz eingeführten Herzkatheters 30 mit Elektrodenabschnitten 31a bis 31d zur Erfassung eines intrakardialen EKG bzw. des Startpunktes für die natürliche AV-Überleitungszeit und sechs gleichartigen Druckaufnehmern 32a bis 32f zur Erfassung eines der oben erwähnten Triggerpunkte und Fig. 3b zeigt Darstellungen von zeitlichen Signalverläufen der in Fig. 3a dargestellten Druckaufnehmer im Vergleich mit dem Signalverlauf eines parallel (mittels einer nicht gezeigten EKG-Anordnung) aufgenommenen Oberflächen-EKG.

Die Druckaufnehmer 32a bis 32f sind in einer sich vom klappennahen Bereich des Vorhofes bis in die Herzspitze erstreckenden Reihung angeordnet, d.h. mit unterschiedlichem Abstand zur Trikuspidalklappe.

Über die Elektroden 31a bis 31d können Stimulationsimpulse angelegt und zudem in differential bipolarer Weise Herzaktionen im Atrium erfaßt werden. In der Figur sind als hierfür vorgesehene Teile einer Verarbeitungseinheit 33 ein mit den Elektroden 31a und 31d verbundener Differenzverstärker 33.1 und eine mit dessen Ausgang verbundener Elektrogramm-Auswertungseinheit 33.2 dargestellt. Eine gute Trennung zwischen antegradem und retrogradem Vorhofsignal ist mit geeigneten Eingangsfiltern möglich. Durch geeignete Ausführung der Signalerfassung kann ein Fehlsensing praktisch ebenso ausgeschlossen werden wie die Hervorrufung schrittmacherinduzierter Tachykardien.

Die Druckaufnehmer 32a bis 32f liefern mit zunehmendem Abstand von der Trikuspidalklappe gegeneinander zeitlich versetzte Signale mit sehr ähnlicher Signalform, wie Fig. 3b in den Zeilen 1 bis 6 (entsprechend den Druckaufnehmern 32a bis 32f) erkennen läßt. Dies bietet die Möglichkeit der oben skizzierten Auswertung in einer durch den atrailen Impulserzeuger 17 (Fig. 1) getriggerten Drucksignal-Auswertungseinheit 33.4 nach Separation von Bewegungsanteilen in einer Filterbaugruppe 33.3.

Die Ausgänge der Drucksignal-Auswertungseinheit 33.4 und der Elektrogramm-Auswertungseinheit 33.2 sind mit einer AV-Berechnungseinheit 33.5 zur Berechnung der AV-Verzögerung verbunden, deren Ausgang mit der Steuereinheit 2 des Schrittmachers (Fig. 1) verbunden ist.

Wie Fig. 3a erkennen läßt, nimmt der Herzkatheter 30 in vorteilhafter Weise in einem einzigen (wie im ovalen Fenster in der Figur zu erkennen), als Doppelleitung ausgeführten) Strang alle Leitungen zur Bestimmung der AV-Verzögerung und zur Stimulation im Atrium auf. Er ist durch geeignet steife Ausbildung der Leiter und/oder Isolation und ggfs. Einprägung einer zusätzlichen vorgegebene Krümmung mechanisch so ausgebildet, daß er keinen Kontakt mit den Herzwandungen hat.

Die Druckaufnehmer 32a bis 32f weisen in einer vorteilhaften Ausführung elastisch verformbare Druckflächen bzw. Membranabschnitte an der Katheteroberfläche mit je einem diesen verformbaren Abschnitten zugeordneten Fühler auf, wobei grundsätzlich die weiter oben genannten Fühlertypen zum Einsatz kommen können.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch in anders gearteten Ausführungen Gebrauch machen.

**Patentansprüche**

1. Vorrichtung zur Bestimmung der AV-Überleitungszeit bzw. AV-Verzögerung im Herzen, mit einer Signalaufnehmeranordnung (EA, EV, P; 31a-d, 32af), welche im Herzen angeordnet werden kann und mit der eine natürliche Herzaktion erfasst werden kann, mit mindestens einem Signalausgang und einer Verarbeitungseinheit (2; 33) mit mindestens einem Signaleingang, der mit dem Signalausgang der Signalaufnehmeranordnung verbunden ist, zur Berechnung der natürlichen AV-Überleitungszeit aufgrund der erfassten Herzaktion, wobei die Signalaufnehmeranordnung eine Druck- bzw. Bewegungsaufnehmeranordnung (P; 32a-f) zur Erfassung von Bewegungen der Trikuspidalklappe und/oder intrakardialer Druckänderungen als Ausdruck der Herzaktion aufweist welche benachbart zur Trikuspidalklappe im rechten Vorhof und/oder in der rechten Herzkammer angeordnet werden kann, **dadurch gekennzeichnet, daß** die Druckaufnehmeranordnung (32a-f) mehrere Druck- bzw, Beweaungsaufnehmer umfasst, welche in verschiedenen Positionen relativ zur Trikuspidalklappe angeordnet werden können und die jeweils einen Signalausgang aufweisen, und dass die Verarbeitungseinheit (33) eine der Anzahl der Druckaufnehmer entsprechende Anzahl von Signaleingängen sowie Mittel zur Auswertung (33.4) der von den mehreren Druck- bzw. Bewegungsaufnehmern empfangenen Signale unter Berücksichtigung ihrer jeweiligen zeitlichen Verzögerung aufweist.

2. Vorrichtung nach Anspruch 1, **dadurch gekennzeichnet, dass** mindestens ein Druckaufnehmer (32a, 32b) im Vorhof und mindestens einer (32c-f) in der Herzkammer angeordnet werden kann.

3. Vorrichtung nach einem der vorangehenden Ansprüche, **dadurch gekennzeichnet, dass** der Verarbeitungseinheit Fühlmittel (EA, 2) zur Erfassung eines spontanen oder induzierten Vorhofsignals mit einem Signalausgang zugeordnet sind, der mit einem entsprechenden Signaleingang der Verarbeitungseinheit (2; 33)verbunden ist, und dass sie Zeitgebermittel und Schaltmittel zur Sperrung des mit der Druckaufnehmeranordnung verbundenen Signaleingangs zur Vorgabe eines Zeitfensters für die Erfassung der Eingangssignale der Druckaufnehmeranordnung (P; 32a-f) aufweist.

4. Vorrichtung nach einem der vorangehenden Ansprüche, **dadurch gekennzeichnet, dass** die Verarbeitungseinheit (33) Mittel (33.3) zur Diskriminierung von Bewegungs- und Druckanteilen im Signal des Druck- bzw. Bewegungsaufnehmers (32a-f), insbesondere mindestens ein niederfrequentes Bandpassfilter, aufweist.

5. Vorrichtung nach einem der vorangehenden Ansprüche, **dadurch gekennzeichnet, dass** die Druckaufnehmeranordnung (P; 32a-f) auf einem Herzkatheter (K; 30) angeordnet ist.

6. Vorrichtung nach einem der vorangehenden Ansprüche, **dadurch gekennzeichnet, dass** die Druckaufnehmeranordnung einen die Bewegung oder Verformung einer Druckaufnahmefläche auf elektrischem Wege erfassenden Sensor, insbesondere einen piezoelektrischen, kapazitiven oder als Dehnungsmeßstreifen wirkenden Drucksensor, aufweist.

7. Vorrichtung nach einem der vorangehenden Ansprüche, **dadurch gekennzeichnet, dass** die Druckaufnehmeranordnung einen die Bewegung oder Verformung einer Druckaufnahmefläche auf optischem Wege, insbesondere durch Ablenkung oder Änderung der Transmission eines auf die Fläche gerichteten, faseroptisch zum Messort übertragenen Lichtbündels, erfassenden Sensor aufweist.

8. Vorrichtung nach Anspruch 6 oder 7, **dadurch gekennzeichnet, dass** auf dem Herzkatheter (30) mehrere, jeweils durch voneinander getrennte Mantelflächenabschnitte des Herzkatheters gebildete oder mit solchen verbundene, Druckaufnahmeflächen (32a-f) angeordnet sind.

9. Vorrichtung nach einem der Ansprüche 5 bis 8, **dadurch gekennzeichnet, daß** der Herzkatheter (K; 30) mit einer solchen Biegesteifigkeit aufgebaut und ggfs. solchen vorgeprägten Krümmung im Längsverlauf versehen ist, dass er im wesentlichen ohne Kontakt mit den Herzinnenwandungen freiliegend im Vorhof und/oder der Herzkammer angeordnet werden kann.

10. Vorrichtung nach einem der vorangehenden Ansprüche, **dadurch gekennzeichnet, dass** mindestens eine Elektrode (EA; 25 31a-d) zur Aufnahme eines Herzaktionspotentials im Herzen vorgesehen ist, die mit einem entsprechenden Signaleingang der Verarbeitungseinheit (2; 33) verbunden ist, und dass die Verarbeitungseinheit Mittel (33.5) zur Bestimmung des Zeitabstandes zwischen einem aus dem Herzaktionspotential abgeleiteten und einem von der Druck- bzw. Bewegungsaufnehmeranordnung (P; 32a-f) gelieferten Signal aufweist.

11. Vorrichtung nach einem der vorangehenden Ansprüche, **dadurch gekennzeichnet, daß** eine Vorrichtung (17, EA) zur Erzeugung eines Reizimpulses und zu dessen Übertragung an den Vorhof vorgesehen ist, die einen Signalausgang aufweist, der mit einem entsprechenden Signaleingang der Verarbeitungseinheit (33) verbunden ist, und daß die Verarbeitungseinheit Mittel (33.5) zur Bestimmung des Zeitabstandes zwischen einem abgegebenen Reizimpuls und einem von der Druck- bzw. Bewegungsaufnehmeranordnung (P; 32a-f) gelieferten Signal aufweist.

12. Herzschrittmacher mit einer Vorrichtung nach einem der vorangehenden Ansprüche, **dadurch gekennzeichnet, dass** der Signalausgang der Verarbeitungseinheit (33) mit einem Signaleingang einer Steuereinheit (2) zur zeitnahen Programmierung einer AV-Verzögerungszeit des Schrittmachers mit dem jeweils zuletzt durch die Verarbeitungseinheit ausgegebenen Wert verbunden ist.

**Claims**

1. Apparatus for determining the AV transmission time or AV delay in the heart, comprising a signal sensor arrangement (EA, EV, P; 31a-d, 32af) which can be arranged in the heart and with which a natural cardiac action can be detected, with at least one signal output and a processing unit (2; 33) with at least one signal input connected to the signal output of the signal sensor arrangement, for calculating the natural AV transmission time on the basis of the detected cardiac action, wherein the signal sensor arrangement has a pressure or motion sensor arrangement (P; 32a-f) for detecting movements of the tricuspid valve and/or intracardial changes in pressure as an expression of the cardiac action, which can be arranged adjacent to the tricuspid valve in the right atrium and/or the right ventricle, **characterised in that** the pressure sensor arrangement (32a-f) includes a plurality of pressure or motion sensors which can be arranged in various positions relative to the tricuspid valve and which each have a respective signal output and that the processing unit (33) has a number of signal inputs, which corresponds to the number of pressure sensors, and means (33.4) for evaluating the signals received from the plurality of pressure or motion sensors having regard to the respective time delay thereof.

2. Apparatus according to claim 1 **characterised in that** at least one pressure sensor (32a, 32b) can be arranged in the atrium and at least one (32c-f) can be arranged in the ventricle.

3. Apparatus according to one of the preceding claims **characterised in that** associated with the processing unit are sensing means (EA, 2) for detecting a spontaneous or induced atrium signal having a signal output which is connected to a corresponding signal input of the processing unit (2; 33) and that it has timing means and switching means for blocking the signal input connected to the pressure sensor arrangement, for presetting a time window for detecting the input signals of the pressure sensor arrangement (P; 32a-f).

4. Apparatus according to one of the preceding claims **characterised in that** the processing unit (33) has means (33.3) for discriminating motion and pressure components in the signal of the pressure or motion sensor (32a-f), in particular at least one low-frequency band pass filter.

5. Apparatus according to one of the preceding claims **characterised in that** the pressure sensor arrangement (P; 32a-f) is arranged on a cardiac catheter (K; 30).

6. Apparatus according to one of the preceding claims **characterised in that** the pressure sensor arrangement has a sensor for electrically detecting the movement or deformation of a pressure sensing surface, in particular a piezoelectric or capacitive pressure sensor or a pressure sensor which acts as a strain gauge.

7. Apparatus according to one of the preceding claims **characterised in that** the pressure sensor arrangement has a sensor detecting the movement or deformation of a pressure sensing surface optically, in particular by deflection of or alteration of the transmission of a light beam which is directed on to the surface and which is transmitted by fibre optic means to the measurement location.

8. Apparatus according to claim 6 or claim 7 **characterised in that** arranged on the cardiac catheter (30) are a plurality of pressure sensing surfaces (32a-f) which are respectively formed by mutually separate peripheral surface portions of the cardiac catheter or are connected to same.

9. Apparatus according to one of claims 5 to 8 **characterised in that** the cardiac catheter (K; 30) is constructed with such a flexural stiffness and optionally provided with such a pre-imposed curvature in the longitudinal configuration that it can be arranged in a freely disposed condition substantially without contact with the inside walls of the heart in the atrium and/or the ventricle.

10. Apparatus according to one of the preceding claims **characterised in that** there is provided at least one electrode (EA; 25, 31a-d) for sensing a cardiac action potential in the heart, which is connected to a corresponding signal input of the processing unit (2; 33), and that the processing unit has means (33.5) for determining the time interval between a signal derived from the cardiac action potential and a signal supplied by the pressure or motion sensor arrangement (P; 32a-f).

11. Apparatus according to one of the preceding claims **characterised in that** there is provided a device (17, EA) for producing a stimulation pulse and for the transmission thereof to the atrium, having a signal output connected to a corresponding signal input of the processing unit (33), and that the processing unit has means (33.5) for determining the time interval between a delivered stimulation pulse and a signal supplied by the pressure or motion sensor arrangement (P; 32a-f).

12. A cardiac pacemaker having an apparatus according to one of the preceding claims **characterised in that** the signal output of the processing unit (33) is connected to a signal input of a control unit (2) for real time-close programming of an AV delay time of the pacemaker with the respective value last outputted by the processing unit.

**Revendications**

1. Dispositif pour la détermination du temps de transition AV ou du retard AV dans le coeur, comportant un dispositif de captage de signaux (EA,EV,P;31a-d,32af) qui peut être disposé dans le coeur, et au moyen duquel une action cardiaque naturelle peut être détectée, et comportant au moins une voie de transmission de signaux et une unité de traitement (2;33) comportant au moins une entrée de signaux, qui est reliée à la sortie de signaux du dispositif de captage de signaux, pour calculer le temps de transfert AV naturel de l'action cardiaque détectée, dans lequel le dispositif d'enregistrement de signaux comporte un dispositif d'enregistrement de pression ou de déplacement (P;32a-f) servant à détecter des déplacements de la valve tricuspide et/ou des variations de pression intracardiaques en tant qu'expression de l'action cardiaque, et qui peut être disposé au voisinage de la valve tricuspide dans l'oreillette droite et/ou dans la chambre cardiaque de droite, **caractérisé en ce que** le dispositif d'enregistrement de pression (32a-f) comprend plusieurs enregistreurs de pression ou de déplacement, qui peuvent être disposés dans différentes positions par rapport à la valve tricuspide et qui possèdent chacun une sortie de signaux, et que l'unité de traitement (33) possède un nombre d'entrées de signaux, qui correspond au nombre d'enregistreurs de pression, ainsi que des moyens pour évaluer (33.4) les signaux reçus de la part de la pluralité d'enregistreurs de pression ou de déplacement, en tenant compte de leurs retards respectifs.

2. Dispositif selon la revendication 1, **caractérisé en ce qu'**au moins un enregistreur de pression (32a, 32b) peut être disposé dans l'oreillette et au moins un enregistreur de pression (32c-f) peut être disposé dans la chambre cardiaque.

3. Dispositif selon l'une des revendications précédentes, **caractérisé en ce qu'**à l'unité de traitement sont associés des moyens de détection (EA,2) servant à détecter un signal spontané ou induit de l'oreillette avec une sortie de signaux et comportant une sortie de signaux, qui est reliée à une entrée correspondante de signaux de l'unité de traitement (2;33) et que ces moyens possèdent des moyens d'indication de temps et des moyens de commutation pour bloquer l'entrée des signaux, qui est reliée au dispositif d'enregistrement de pression, pour la délivrance d'une fenêtre temporelle pour la détection des signaux d'entrée du dispositif (P;32a-f) d'enregistrement de pression.

4. Dispositif selon l'une des revendications précédentes, **caractérisé en ce que** l'unité de traitement (33) comporte des moyens (33.3) pour discriminer des composantes de déplacement et de pression dans le signal de l'enregistreur de pression ou de déplacement (32a-f), notamment au moins un filtre passe-bande à basse fréquence.

5. Dispositif selon l'une des revendications précédentes, **caractérisé en ce que** le dispositif d'enregistrement de pression (P;32a-f) est disposé sur un cathéter cardiaque (K;30).

6. Dispositif selon l'une des revendications précédentes, **caractérisé en ce que** le dispositif d'enregistrement de pression comporte un capteur, qui détecte le déplacement ou la déformation d'une surface de captage de pression, par des moyens électriques, notamment un capteur de pression piézoélectrique, capacitif ou agissant en tant que jauge extensométrique.

7. Dispositif selon l'une des revendications précédentes, **caractérisé en ce que** le dispositif d'enregistrement de pression comporte un capteur, qui détecte le déplacement ou la déformation d'une surface d'enregistrement de pression par voie optique, notamment par déviation et modification de la transmission d'un faisceau de lumière dirigé vers la surface et transmis par fibre optique à l'emplacement de mesure.

8. Dispositif selon la revendication 6 ou 7, **caractérisé en ce que** sur le cathéter cardiaque (30) sont disposées plusieurs surfaces d'enregistrement de pression (32a-f) formées respectivement par des sections séparées les unes des autres de la surface enveloppe du cathéter cardiaque ou reliées à de telles sections de surface.

9. Dispositif selon l'une des revendications 5 à 8, **caractérisé en ce que** le cathéter cardiaque (K;30) est constitué avec une résistance à la flexion telle et est pourvue éventuellement, dans la direction longitudinale, d'une courbure préappliquée telle qu'il peut être disposé librement essentiellement sans contact avec les parois intérieures du coeur dans l'oreillette et/ou dans la chambre cardiaque.

10. Dispositif selon l'une des revendications précédentes, **caractérisé en ce qu'**il est prévu une électrode (EA; 25, 31a-d) pour l'enregistrement d'un potentiel d'action cardiaque dans le coeur, qui est reliée à une entrée correspondante de signaux de l'entrée de traitement (2;33) et que l'unité de traitement comporte des moyens (33.5) pour déterminer l'intervalle de temps entre un signal dérivé du potentiel d'action cardiaque et un signal délivré du dispositif (P;32a-f) d'enregistrement de pression ou de déplacement.

11. Dispositif selon l'une des revendications précédentes, **caractérisé en ce qu'**il est prévu un dispositif (17;EA) servant à produire une impulsion d'excitation et à transmettre cette dernière à l'oreillette et qui comporte une sortie de signaux, qui est reliée à une entrée correspondante des signaux de l'unité de traitement (33), et que l'unité de traitement comporte des moyens (33.5) pour déterminer l'intervalle de temps entre une impulsion d'excitation délivrée et un signal délivré par le dispositif d'enregistrement de pression ou de déplacement (P; 32a-f).

12. Stimulateur cardiaque comportant un dispositif selon l'une des revendications précédentes, **caractérisé en ce que** la sortie de signaux de l'unité de traitement (33) est reliée à une entrée de signaux de l'unité de commande (2) pour la programmation actuelle d'un temps de retard AV du stimulateur, avec la valeur respectivement délivrée en dernier lieu par l'unité de traitement.

EP 0 865 802 B1

Fig.1

Fig.2

Fig.3a

Fig.3b